# EUROPEAN PATENT APPLICATION

(11) **EP 1 693 072 A1**
(43) Date of publication of application: **23.08.2006**
(21) Application number: 06006410.2
(22) Date of filing: 28.04.2003
(51) Int. Cl.: A61L 2/08, A61M 1/36, A61L 2/26, B01F 11/00, B01F 9/00, A61L 2/16

(54) **Apparatus and method for irradiating and mixing fluids in containers**

(30) Priority: 26.04.2002 US 375734 P
(62) Divisional of application: 03726521.2
(71) Applicant: Gambro, Inc.,, Lakewood Colorado 80215 (US)
(72) Inventor: Hlavinka, Dennis J., Arvada Colorado 80007 (US); Martinez, Michael A., Golden Colorado 80403 (US)
(74) Representative: Roberts, Mark Peter

(57) **Abstract**

A mixing system, apparatus and/or method. Pathogen reduction of and/or mixing storage solutions into blood or blood components are useful purposes for these. Squeezing or clapping devices may be used to activate the mixing process, as may rotational devices or laterally movable, rotational and/or orbital devices. Constriction elements may also be used to create useful vortex mixing actions. Photoradiation may be provided while the components continue to be mixed together for pathogen reduction of blood or blood components.

## Description

This application claims priority from United States Provisional application 60/375734, filed April 26, 2002.

### FIELD OF THE INVENTION

This invention is generally related to apparatuses and/or methods for mixing the contents of various containers or bags while the contents are being irradiated. Of particular note is the use of these apparatuses and methods in a pathogen reduction procedure.

### BACKGROUND

Contamination of human blood and blood components with pathogens such as human immunovirus (HIV), hepatitis and/or bacteria create a serious risk for patients who receive blood or blood components via blood transfusions.

To help combat the problem of pathogenic contamination in blood and/or blood components, one method of reducing pathogens in blood and biologically useful fluids may be to use radiation to substantially destroy any pathogens contained in the fluid. Radiation may be used to inactivate pathogens contained in blood and blood components by generating mutagenic alterations in their genetic material. Above a minimum dose of radiation, the pathogens lose their capacity to reproduce. Radiation damages the nucleic acids of the pathogens by creating intrastrand nicks and inducing nucleotide photodimerization, both of which disrupt nucleic acid replication. Through such mechanisms, irradiating blood and blood components with either visible or ultraviolet (UV) light can be an effective means for reducing undesirable pathogens within blood and other biologically useful fluids.

Unfortunately, the energy of short wavelength UV light may also damage the blood and blood components that are the desired end-products of the irradiation process. Thus, an inherent problem in the application of UV-irradiation techniques is controlling the irradiation of the fluid so as to ensure sufficient radiation exposure to reduce undesirable pathogens within a fluid while at the same time minimizing or eliminating damage to the biologically useful fluids. One way to avoid substantial damage to the biologically useful fluid by UV light is to design an apparatus which will effectively mix the fluid in such as way so as not to over-expose the fluid to the radiation.

Blood and blood components can also be decontaminated using pathogen reducing agents or photosensitizers which, when activated, also reduce pathogens contained in the blood or other biologically useful fluids but does not destroy the biological activity of the blood or blood component product.

Pathogen reduction agents, which may be used with this invention, include the class of photosensitizers known in the art to be useful for reducing pathogens. A "photosensitizer" as defined here is any compound which absorbs radiation of one or more defined wavelengths and subsequently transfers the absorbed energy to an energy acceptor. Thus, such photosensitizers may be activated by the application of electromagnetic spectra (e.g., UV and visible light) to then reduce certain pathogens with which they may interact.

Various photosensitizers have been proposed for use as blood or blood component additives to inactivate pathogens in body fluids. Examples of non-endogenous photosensitizers that have been proposed for use as blood or blood component additives include porphyrins, psoralens, acridines, toluidines, flavins (acriflavin hydrochloride), phenothiazine derivatives, coumarins, quinolines, quinones, anthroquinones and dyes such as neutral red and methylene blue.

Other categories of photosensitizers are endogenous pathogen reduction agents, such as 7,8,10-trimethylisoalloxazine (lumiflavin), 7,8-dimethylalloxazine (lumichrome), isoalloxazine-adenine dinucleotide (flavin adenine dinucleotide [FAD]), alloxazine mononucleotide (flavin mononucleotide [FMN] and riboflavin-5-phosphate), vitamin K and vitamin L and their metabolites and precursors, napththoquinones, naphthalenes and naphthols as well as their derivatives. One preferred example of an endogenous photosensitizer contemplated for use with this invention is an alloxazine such as 7,8-dimethyl-10-ribityl isoalloxazine, commonly known as riboflavin. An advantage of using endogenous photosensitizers to reduce blood contaminants is that endogenous photosensitizers are not inherently toxic to the blood cells and if photoactivated do not yield toxic photoproducts after exposure to radiation. Therefore, a removal or purification step is not required after the decontamination process, and the treated product can then be stored in the same solution used in the pathogen reduction process, transfused into a patient, or returned directly to the donor.

One method of decontaminating blood or blood components using a photosensitizer includes mixing an effective amount of a photosensitizer with the fluid to be decontaminated in a batch-wise way; then exposing the fluid to an amount of photoradiation at an appropriate wavelength sufficient to activate the photosensitizer and allow the activated agent to interfere with the pathogens contained within the fluid such that the pathogens contained in the fluid are reduced. The wavelength of light used will depend on the photosensitizing agent selected as well as the type of blood components being pathogen reduced. The light source or sources may provide light in the visible range, the ultraviolet range, or a mixture of light in both the visible and the ultraviolet range

Decontamination or pathogen reduction systems may be designed as stand-alone units as described above, or may be incorporated into existing apparatuses known to the art for separating or treating blood to be withdrawn from or administered to a patient. For example, such blood-handling apparatuses include the COBE Spectra™ or TRIMA® apheresis systems, available from Gambro BCT Inc., Lakewood, Colorado, as well as apheresis systems of other manufactures. The decontamination system may be inserted before the collected blood is separated into components. Alternatively, the decontamination system may be inserted downstream of the point where the blood is separated and/or collected, or at any point after separation of blood constituents. It may even be inserted just prior to reinfusion of the blood product back into the patient. It is further understood that discrete irradiation sources could be placed upstream from the collection points of each separated blood component, such as red blood cells, platelets, and plasma. The use of three separate blood decontamination systems, one for each separated blood component, may be preferred to placement of a single blood decontamination system upstream of the blood separation vessel of an apheresis system because the lower flow rates in the separated component lines may allow for greater ease of irradiation.

In other embodiments, decontamination systems for use in and/or with the present invention may be used to process previously collected and/or stored blood products, whole blood or components, in a batch-wise way, as discussed above, and in further detail below. In some photosensitizer methods, the blood product to be decontaminated is flowed through an entry port into a photopermeable bag or other container. The term "photopermeable" means that the material of the container is adequately transparent to photoradiation.

Polymeric bags and like containers, flexible or otherwise, which are commonly used to collect and store blood and blood components, are useful as the photopermeable containers referred to above.

After the pathogen reduction process, the pathogen reduced fluid may then be flowed out of the photopermeable container into a storage container through an exit port, or may be stored in the same photopermeable container used in the photoinactivation process until transfused into a patient.

One problem with the use of light alone or light in combination with a photosensitizer to reduce pathogens in blood or blood products, is that during the pathogen reduction process, a portion of the fluid to be pathogen reduced may become trapped within dead spaces or opaque portions of the bag or container. Fluid trapped in these dead spaces or opaque portions may not be reached by light and may therefore still contain pathogens which will re-infect the fluid which was previously pathogen reduced.

Another problem in pathogen reducing fluid using light results from the laminar nature of fluid flow in a container. In either a flow-through or a batch wise system, a parabolic velocity profile exists for the fluid contained in either the fluid-flow channel or a self contained bag. Upon agitation or application of a force, the fluid at the center of the flow channel or bag is traveling at a maximum velocity, while the fluid close to the walls at the bag-fluid interface remains nearly stationary. Because of this flow profile, upon irradiation of blood or blood components, the exposure time of the blood is the shortest for the blood traveling at maximum velocity at the center of the container, and increases for successive portions of the flow profile moving outwardly from the center. Therefore, not all of the blood in a bag is irradiated at the same intensity and for the same length of time. In addition to the velocity profile, blood tends to spread in a thin film along the surface of the bag due to surface tension and the tendency of blood to cling to the bag's surface. In the absence of vigorous agitation, the blood located along the walls of the container would have an extremely long residence time. Thus, the blood or blood component nearest the walls (closest to the irradiation source) runs the risk of being overexposed to radiation which may significantly damage the blood or blood components, while the fluid in the middle of the container runs the risk of being under irradiated, thus any pathogens contained in this region would receive little or no radiation, and would be likely to re-contaminate the fluid with still viable pathogens.

In view of the above background, it can be seen that there is a need for a method and apparatus for pathogen reducing a fluid that ensures adequate exposure of all of the fluid to radiation while simultaneously minimizing the damage to the blood or blood components.

### SUMMARY OF THE INVENTION

The present invention relates to methods, systems and apparatuses for mixing various fluid (or other substance) elements. More particularly, the present invention applies desirably to the preparation including mixing of a blood or blood component product in a pathogen reduction procedure. In one embodiment, the apparatus comprises a support structure for hanging a flexible polymeric container or bag therein or thereon and a moveable "clapper" structure which alternately squeezes the bag and releases the bag to mix the contents thereof. A clamp-like structure may also be provided to create one or more constrictions in the bag to provide mixing vortices within the bag to enhance the exposure of the bag contents to or adjacent the internal surface of the bag and thus also to any photoradiation impinging thereon. Photoinactivation is thus enhanced particularly for relatively opaque fluids (such as RBCs). A photosensitizer may be added to the blood or blood component to be pathogen inactivated and then thoroughly mixed therein. Then, also or alternatively the mixing effect could be sustained during illumination of the product therein.

Another embodiment involves a rotating structure in which a flexible bag may be disposed such that when rotated, the contents of the bag are continually rotated up and alternately pulled down by gravity. Rotating embodiments may also have light illumination and/or clamp-like structures constricting portions of the bag to create vortices which enhance mixing.

### BRIEF DESCRIPTION OF THE DRAWINGS

Fig. 1 is a schematic isometric view of one embodiment of a mixing system according to the present invention.

Fig. 2 is another isometric view of an embodiment of a mixing system according to the present invention.

Fig. 3 shows an isometric view of a flexible container and a mixing assembly according to one embodiment of the present invention.

Fig. 4 shows a substantially elevational view of a flexible container and a mixing assembly according to the embodiment of Fig 3.

Fig. 5 illustrates an extracorporeal tubing and bag assembly which may be used with and/or in a system of the present invention.

Fig. 6 shows a partial cross-sectional view of a bag and clamp assembly.

Fig. 7 shows a partial cross-sectional view of a bag and clamp assembly as in Fig. 6.

Fig. 8 shows another partial cross-sectional view of a bag and clamp assembly as in Fig. 6.

Fig. 9 shows an elevational schematic view of a flexible container and clamp assembly.

Fig. 10 shows an isometric view of an alternative mixing assembly.

Fig. 11 shows a plan view of the alternative embodiment of a mixing structure according to Fig. 10.

Fig. 12 shows an elevational view of another embodiment of a mixing structure and clamp.

Fig. 13 shows a partially cut away isometric view of a mixing structure with a flexible container.

Fig. 14 shows an clevational view of an alternative embodiment mixing structure somewhat like that in Figs. 12-13.

Fig. 15 shows an isometric view of an alternative embodiment of a mixing structure according to the present invention.

Fig. 16 shows a side elevational view of the alternative embodiment of Fig. 15.

Fig. 17 shows another side elevational view of the embodiment of Fig. 15.

Fig. 18 shows a rear isometric view of the embodiment of Fig. 15.

Fig. 19 is a plan view of a frame for use with the embodiments of Figs. 15-18.

Fig. 20 is an isometric view of an alternative embodiment of the present invention.

Fig. 21 is a top plan view of the embodiment of Fig. 20.

Fig. 22 is an isometric view of another alternative embodiment of the present invention.

Fig. 23 is an isometric view of a part of the embodiment of Fig. 22.

Fig. 24 shows a partial cross-sectional view including fluid vortices created within a bag used in an embodiment like that shown in Fig. 22 upon application of a force causing the fluid to flow in the general direction of the lateral pointing arrows.

Fig. 25 shows a partial cross-sectional view including fluid vortices created within a bag used in an embodiment like that shown in Fig. 22 upon rotation in the direction of the arrows.

Fig. 26 shows a plan view of an alternative bag for containing fluid to be mixed according to the present invention.

Fig. 27 shows a plan view of an alternative bag for containing fluid to be mixed according to the present invention.

### DETAILED DESCRIPTION

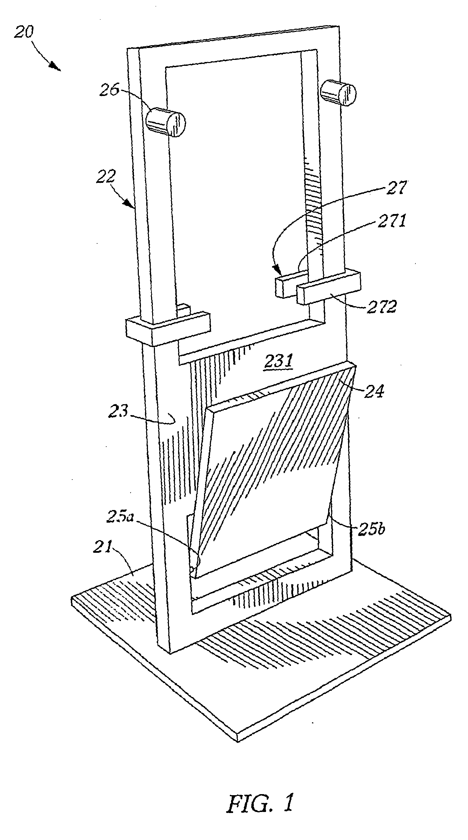

Fig. 1 shows a blood or blood component mixing system 20 for mixing blood components in accordance with the present invention. Whole blood is withdrawn from a donor/patient (not shown) and may be treated in whole blood form by the present invention, or it may be provided to an apheresis system or other type of blood component separation device (not shown) often of a centrifugal type, where the blood may be separated into one or more of various component types and at least one of these blood component types can then be removed/collected as a product from the separation device. The blood or blood component products (e.g., platelets, plasma, white blood cells, or red blood cells) may then be pathogen reduced either continuously in a flow-through manner within or adjacent the apheresis machine (not shown) or in a separate batchwise step. Ultimately, the pathogen reduced blood components may then be stored for later transfusion into a patient.

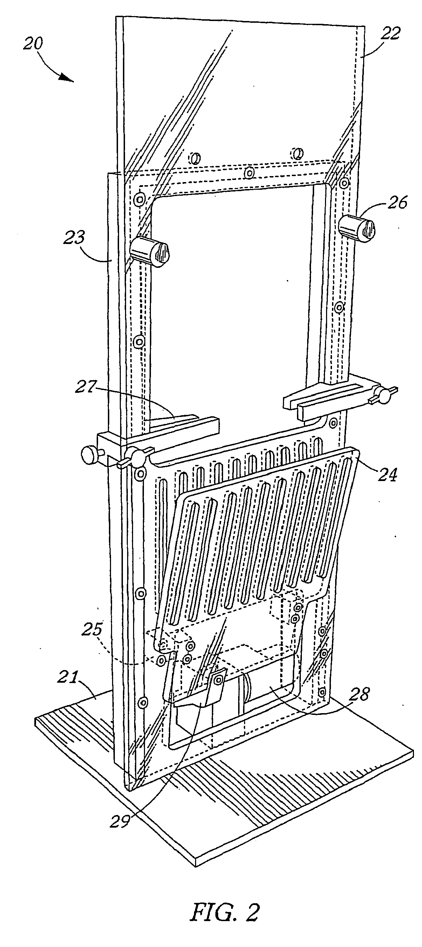

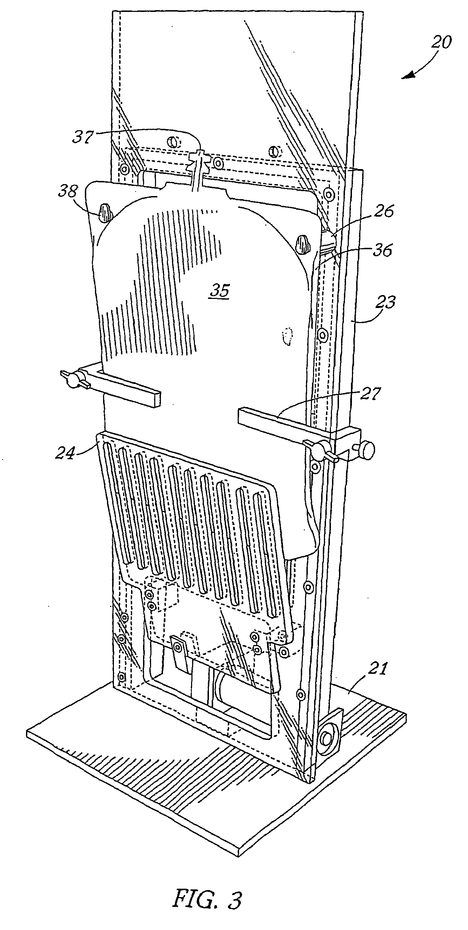

The system **20** shown generally in Fig. 1 includes a mixing device **22** (shown schematically here) which has a support structure generally designated **23** disposed on a base **21.** A moveable "clapping" member **24** is disposed in operative relation to support **23,** and as shown here, has a pivotable relationship therewith as depicted in Fig. 1 by the connection through pins **25** (see pins **25a** and **25b).** As shown in Figs. 2-4, moveable member **24** has openings or slots disposed therein. Such openings or slots may be used to allow radiation to penetrate the bag **35** (see Fig. 3) which may be contained therein, or may be used to allow heat generated during the irradiation process to escape so as not to damage the blood or blood product being irradiated. Support structure **23** also may have, as shown, one or more devices for holding a container therein such as the two holding members **26** which may be, as shown here, protrusions or hooks on which a bag may be hung (see below). Also shown schematically are two clamp-like constriction elements **27,** the use of which for creating mixing vortices will be described further below.

An exemplar device **22** is shown in more detail in Figs. 2-4. One additional detail reflected herein is a motor **28** connected to movable member **24** via a linkage connection **29.** Motor **28** provides the motive force to move the movable member **24** as will be described further below. Fig. 3 also shows a flexible container or bag **35** disposed in/on device **22** as supported by hanging members **26.**

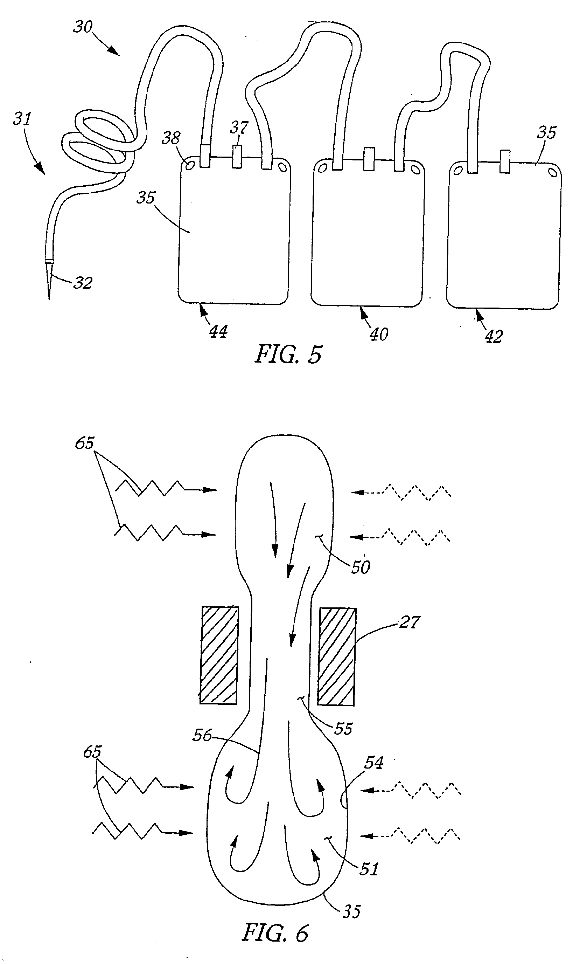

Fig. 5 is but one example of a preconnected extracorporeal tubing circuit **30** which may be used to obtain a blood product according to known (or to be developed) methods. In general, a blood removal/inlet tubing sub-assembly **31** provides a needle **32** to interface a donor (not shown) with the remainder of the tubing circuit **30.** A platelet collection tubing and bag assembly **40,** a plasma collection tubing and bag assembly **42,** and a red blood cell collection tubing and bag assembly **44** may also be connected within circuit **30.** As will be appreciated, this is but one example of an extracorporeal tubing circuit **30** and further various blood component assemblies (more or less than shown) are or may be pre-interconnected to combinatively yield a closed, pre-sterilized disposable assembly suitable for a one time use. Any of these bags **35** may then be used in system **20,** as well as any of the other systems described herein below.

Most portions of the tubing assemblies **30, 40, 42** and **44** including bags **35** may be made from flexible polymeric or plastic components including, for example, polyolefin or polyvinyl chloride (PVC) tubing lines and bags **35,** that preferably permit visual observation and monitoring of blood/blood components therewithin during use, as well as for irradiation purposes. All tubing lines and bags may be preconnected before sterilization of the entire disposable assembly to assure that maximum sterility of the system is maintained. Thus, bag **35** may be pre-connected and/or post-connectable with the extracorporeal tubing circuit **30** as described relative to Fig. 5 above, or may be used separately, as a stand alone apparatus, neither alternative departing from the spirit and scope of the invention. Note further that alternative resilient or even rigid containers may also be used in some embodiments of the present invention.

In any case, Figs: 3 and 4 show any of the Fig. 5 flexible polymeric containers or bags 35 disposed in a mixing structure **22** of a system **20** in accordance with the present invention. The container or bag **35** may be made of a flexible polymeric type film which is sealed or welded around its outer border zones during manufacture to form pre-formed seals or welds (see weld **36** denoting the circumference of container or bag **35).** The seals or welds **36** create a fluid tight, sealed interior space or main body compartment (not separately shown in Figs. 3-4). Ports or openings **37** allow fluid ingress and/or egress into and/or out of the container or bag 35. As shown in Figs. 3-4, two ports **37** may be located on the same side of the container or bag 35. Such a container or bag **35** could contain one, two or more ports as is generally known. Known types of ports may be used in this invention, including one or more ports having a frangible-type closure mechanism (not shown).

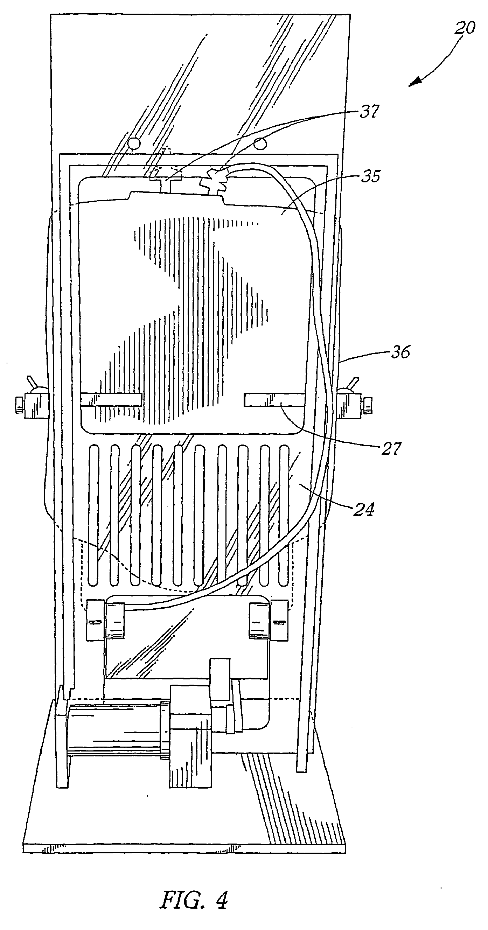

As shown in Fig. 3, the container or bag **35** may have a hole or holes **38** punched in for example, the upper or lower edges of the pre-formed factory seal **36** to mount the container or bag **35** in a hanging position as will be described further. Alternatively, the bag may not have holes punched in the pre-formed factory seal (see Fig. 4) and then may be hung in various other fashions, as for example, using clamps (not shown). The container or bag **35** may be hung before, during or after the process of combining the blood component with any additive solutions, if used, into the bag **35.** The pre-formed factory seal **36** or any of the other pre-formed seals may also be made wide enough so that a label describing the contents of the bag **35** may preferably be placed on the area over the seal (not shown). The bag **35** may also have a connection to a sample bulb via a port (not separately shown) to allow for fluid removal and sample testing or the like. A port which allows for the connection of a spike receptor (not shown) or to enable the sterile docking of a further bag or tube for the addition of a photosensitizer or blood component may also be added to container or bag **35.** A spike connector (not shown) may also preferably include a sterile barrier filter as is known in the art.

In one embodiment, and as introduced above and further described below, an external constriction element or clamp assembly **27** on or otherwise associated with (or dissociated from) device **22** may be a clamp, a clip, or anything of the like which may be removably connected to the container and thus divides a single container into multiple removable sub-compartments by a restricted flow area (though preferably still allowing flow therethrough) and limits fluid communication between each of the separate sub-compartments. More constriction elements than specifically shown in the figures may be used to create more than the two general sub-compartments shown in a single container as well. Alternatively, pre-formed seals or welds (not shown) may be made in container **35** to create one or more sub-compartments with constricted flow areas, yet permitting flow between compartments. Bags containing sub-compartments made with pre-formed seals or welds may be used alone or in combination with clamp assembly **27.** United States Patent Application US2002/0138066, published September 26, 2002 and herein incorporated by reference in its entirety to the amount not inconsistent, discloses containers having multiple pre-formed subcompartments made with seals or welds which may be used with the present invention.

Figs 1-3 show isometric views of clamp assemblies **27** (not shown with container or bag **35** in Figs. 1 and 2, but shown therewith in Fig. 3) according to the present invention. Each clamp assembly **27** preferably includes at least one bar **271** (see Fig. 1), and as shown, preferably a second bar **272.** As will be described, these bars **271, 272** assist in creating a narrowed fluid passage in container **35** between the sub-compartments formed by the clamp assemblies **27.**

Fig. 6 shows a partially broken away cross-sectional view of a clamp assembly **27** (shown in dashed lines) creating separate sub-compartments **50** and **51** in a flexible container or bag 35. The clamp assembly **27** creates a temporary, preferably removable constriction **55** along all or part of the width of the bag to create the two distinct sub-compartments **50** and **51.** The clamp assembly **27** may, in one embodiment, be easily removed from the container or bag 35 (as described below) to create a single bag with only one internal compartment. This may be achieved by simply loosening and/or removing the bar(s) **271, 272** on/from the device **22.** Once the bars **271, 272** are loosened or removed, the bag **35** may be removed from the structure **22** by removing the bag **35** from the hooks **26** (if applicable). In an alternate embodiment, clamp assembly **27** may not need to be removable or loosenable from device **22.** Clamp assembly **27** may be permanently affixed to device **22,** and bag **35** may be slid in and out of the clamp assembly **27.**

One method of using the above-described embodiments is as follows, described in relation to the embodiment of Figs. 1-4 as shown by Figs. 6, 7 and 8. Although not specifically described, the method may also be used generally with the alternative embodiments described below. Initially, blood or blood components to be pathogen reduced are disposed in bag **35.** Any other components which may be desirable for the pathogen reduction procedure such as a photosensitizer may be further disposed in bag **35,** or may be prepackaged in a satellite bag assembly (not shown). Any number of other solutions containing other necessary components for pathogen reduction or other purposes such as storage of blood or separated blood components may be added to bag **35** through port **37** at any desirable time. Note, the additional components may be in a dry solid or a liquid form.

In one use of the invention, solutions for the pathogen reduction and/or storage of blood and/or blood components may be preliminarily mixed together or may be simultaneously mixed with the blood product to be pathogen reduced. After combination of the fluids either before or after addition to the blood or blood component product to be pathogen reduced, the blood or blood component to be pathogen reduced and the solution which may contain a photosensitizer are mechanically mixed together using a mixing structure **22** according to the present invention. The mixture may be exposed to a light source while continuing the mixing process.

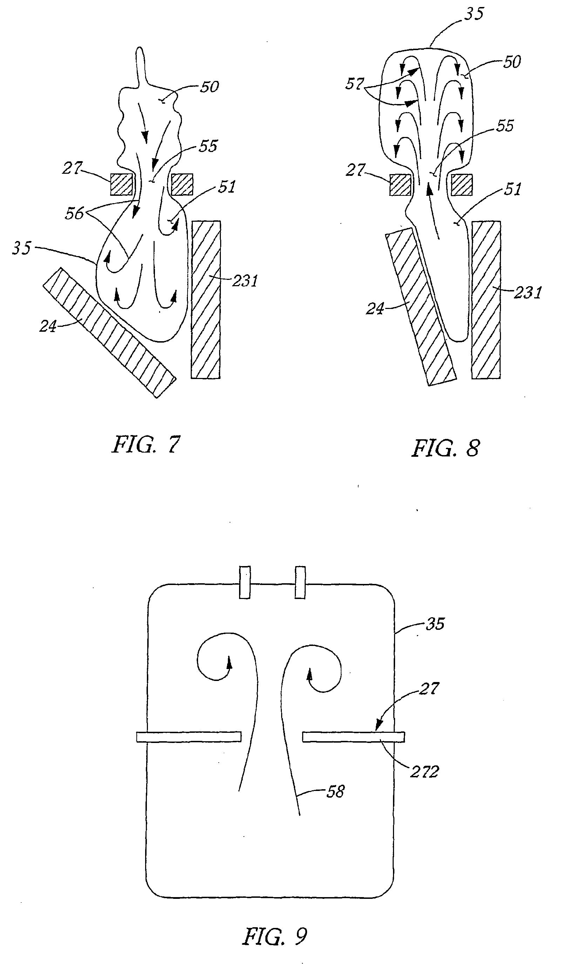

Fig. 6 shows the creation of multiple vortices within a flexible polymeric bag similar to the type described in Figs. 3, 4 and 5, when disposed in operative relationship with a clamping device, such as device **27.** Because of the flexible nature of the bag **35,** clamp **27** causes a partial constriction which may be located approximately halfway up the length of the bag and as shown in Figs. 1-4 each clamp **27** may extend inwardly from the sides of the container/bag **35.** As shown in Figs. 6, 7 and 8, the created narrowed or constricted portion **55** in the sides of the container **35** acts as follows. In the first instance, gravity pulls the fluid down from the upper compartment **50,** through the constriction **55** and then into the lower compartment **51.** This is shown generally in Figs. 6 and 7. Flow vortex action is shown by the flow arrows **56,** which help reach and "scrub" the interior surfaces **54** of the bag **35.** Such mixing action helps to replace the fluid located along the fluid-bag surface interface **54** with fluid from the interior of the bag. Such turn over of fluid helps to prevent overexposure of the fluid nearest the bag surface **54** (and nearest to the irradiation system) to radiation, and helps bring fluid from the interior of the bag **35** to the surface to be irradiated. Although such "scrubbing" action is described in relation to fluid vortices generated by the mixing process, random flow mixing will also work to facilitate fluid turn over. Then, in the second instance, as shown by Fig. 8 and upon the application of a force or pressure to the bag, such as by squeezing of the lower bag portion **51** by the moveable member **24,** the fluid within the container **35** may be forced to move to the upper portion **50** from the lower portion **51** of the container **35** through the narrowed portion **55** created by the clamp(s) **27.** Moveable member **24** is shown in Fig. 7 in a relative open position relative to a back wall **231** (see Fig. 1) of a device **22,** whereas moveable member **24** is shown moved in Fig. 8 to a relative closed position adjacent wall **231** between which the bag 35 has been squeezed thereby providing the force that moved the fluid up from the lower compartment **51** to the upper compartment **50.** This force also caused the flow vortices **57** which scrub the upper inner walls **54** of bag **35.** This movement creates vortices within the fluid as shown in Fig 8 which helps to further mix the fluid. Although a force application and resultant vortices shown in Fig. 8 are shown as being directed towards the upper portion of bag **35** respectively, the force application and resultant vortices could be directed to either the upper portion or the lower portion of the bag **35** or both, depending upon the squeezing or relaxing of the device **22.** In a relatively substantially vertically disposed system **20,** the fluid within the bag **35** may be repeatedly forced to flow to the upper portion **50** of the container by action of device **22** and then allowed to be pulled to the lower portion by gravity to ensure thorough mixing of the blood or blood component product and a photosensitizer (if added) as well as ensuring thorough mixing of the component product to expose the entire product to light during illumination (light rays **65** are shown in Fig. 6). Thus, a substantially vertically disposed device **22** may have an advantage in using gravity for a substantial part of fluid movement, and only a bottom compartment squeezing element may be used.

Such turn over of fluid by mixing provides an advantage especially to pathogen reduction of relatively opaque substances such as RBC component products. With such products, light is not readily able to penetrate very deeply into the component product (e.g. not far past the bag film). Thus, the layer of blood product immediately adjacent the inner bag wall is irradiated, but should then be "turned over" or removed therefrom and replaced with a new layer of blood product which may then be irradiated. Mixing as described herein serves to "scrub" these bag wall layers of product away in desirable fashion. Moreover, such action may be made very aggressive during irradiation and help to shorten the pathogen reduction process.

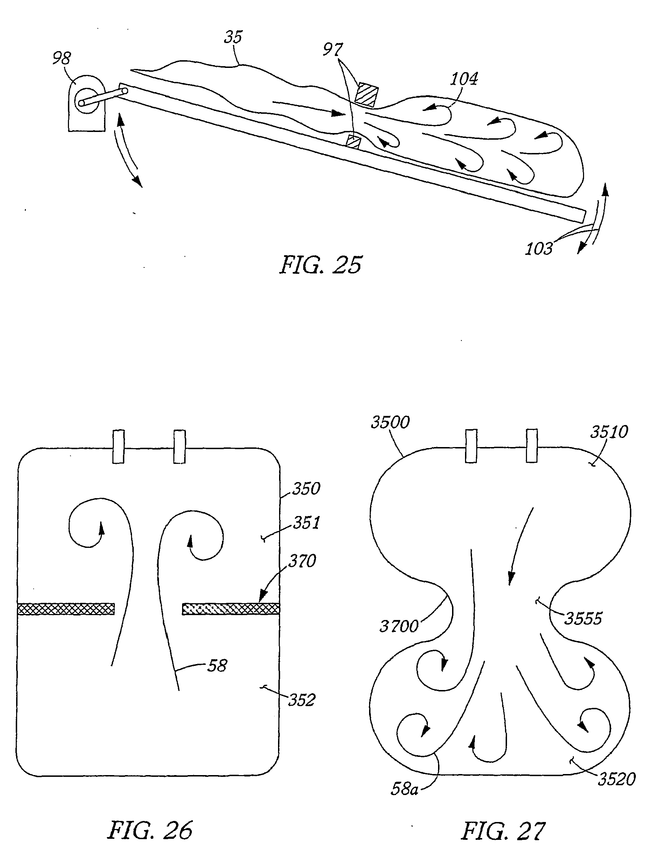

Bag 35 is shown in Figs. 3 and 4 and more particularly in Fig. 9 as constricted by clamps **27** only partially across the width thereof as limited by the lateral extensions of clamps 27. This may provide an advantage because it may provide additional lateral flows and mixing vortices as shown by arrows 58 in Fig. 9. Even so, and although thus far only partial constrictions have been shown and described extending from the outer border zones of the bag 35, it ought to be noted that such a bag could be divided more fully into two (or multiple) sub-compartments by placing one (or more) complete cross bar clamp assemblies over the bag at a desired location similar to the clamp assemblies shown in Figs. 3 and 4, except that they might cover the entire width of the bag 35. Moreover, such a closure assembly could be an external removable openable assembly, an internal openable assembly, a clamp, a clip, or anything of the like which would entirely or partially divide the single container/bag into multiple sub-compartments though continuing to provide merely limited or constricted communication between the fluid contents contained within each separate sub-compartment such that flow is still viable therethrough as shown in Figs. 6, 7 and 8. It is further noted that a number of alternative bag embodiments having one or more partial seals or welds, or other bag shapes such as those shown below in Figs. 26 and 27 (see the hourglass shape therein) could be used as any bag including all of the bags or containers described herein. Otherwise, other mechanical closure (not shown) assemblies or resilient or even rigid containers with built in or external removable assemblies or other structural equivalents such as a clamp, a clip, a tongue and groove seal, a vise, a clasp, a grip, or a fastener may be used to create a narrowed portion within a container. These may be connected to device 22, 72, 92, 400, 500 or not, as may be desired.

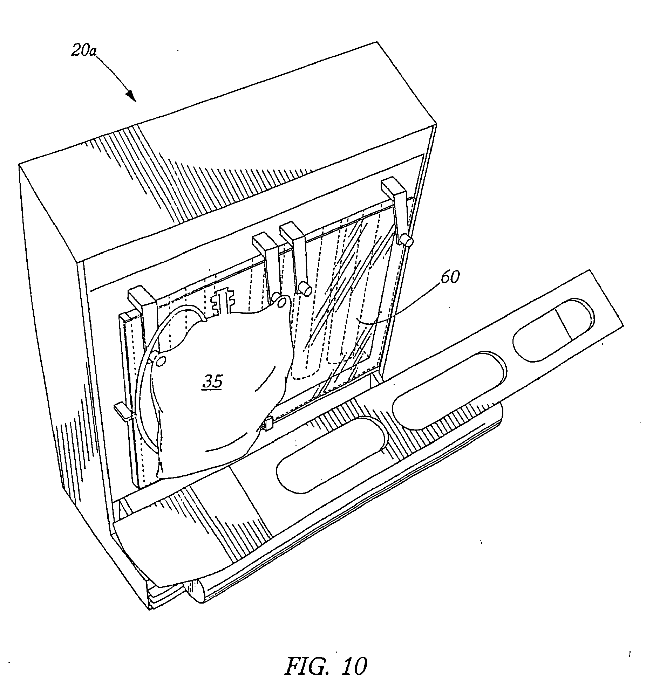

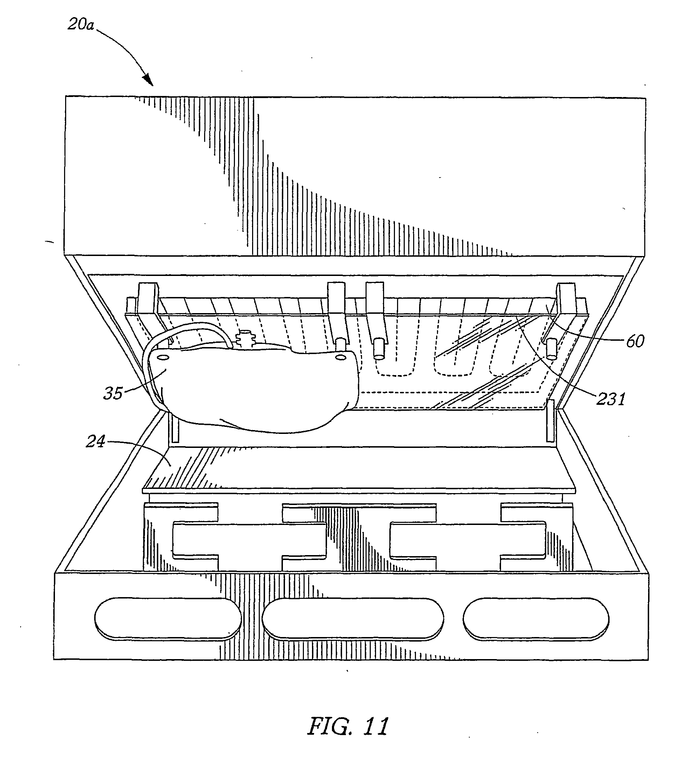

Note, a primary embodiment using a squeezing device such as this could also make use of light sources (not directly shown as yet but see Figs. 10 and 11) to shine on the bag, or multiple bags simultaneously (see the light rays **65** represented schematically in Fig. 6). Such an embodiment with a substantially vertically arranged squeezing or "clapping" mechanism is shown in Figs. 10 and 11. Such a system **20a** is shown having capacity for two bags though only one bag **35** is shown. In this embodiment two full length bars (not shown in Fig. 10) which stretch across the entire width of the two bags could be used one each on the front and back sides of the bags to create the clamping assembly **27** and associated mixing described in detail above. Here, however, at least one set of light sources **60** are shown which can be used to irradiate the bag(s) 35 and the contents thereof while mixing the contents using the motions and vortices described above. The contents may be fully exposed to photoradiation, and if a photosensitizer is used, such mixing would ensure proper exposure of the photoactivatable agent (e.g., riboflavin or psoralens) to the photoradiation, as well as to ensure fluid turn over. Note, though only one set of lights **60** are shown in Figs. 10 and 11, another could be established on the opposing side of the bag(s) **35.** Light radiation arrows **65** are shown in Fig. 6. A reflective surface to reflect the light throughout the chamber could also be used. The dashed line schematic light rays in Fig. 6 are intended to reflect these options (separate light sources or reflective surfaces). In any event, it may be preferred that light be made to impinge upon the bag(s) **35** from both sides (e.g. UV light, if used, may need to be provided from both sides). Note also that it may be advantageous for all or most of the physical elements touching or near bag(s) **35,** e.g., the clapper structure **24** (see Fig. 11) and the back wall **231,** to be transparent to light radiation, and may thus be made of a transparent polycarbonate, plexiglass, quartz glass or other sturdy substantially transparent material to allow light transmissivity therethrough. This may be advantageous in any or all embodiments described herein.

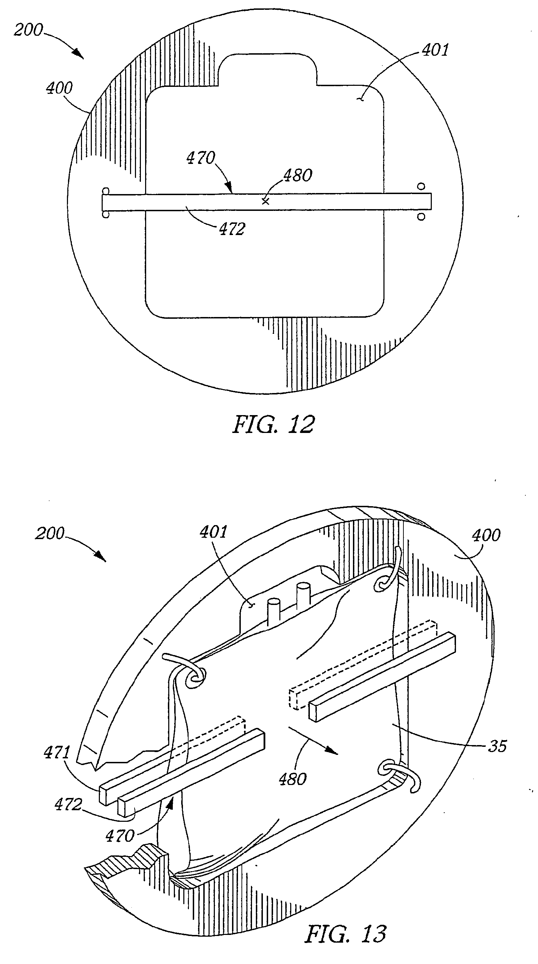

An alternative mixing structure may include a rotatable device or wheel in which a bag may be disposed. Shown in Figs. 12 and 13 is a wheel **400** of a system generally designated **200.** Wheel **400** has a cutout or aperture **401** defined therein in which a bag such as bag 35 (Figs. 3-5) may be disposed. The cutout 401 where the bag 35 may be disposed, may be substantially transmissive to radiation. It is also contemplated that the cutout **401** may not be a cutout at all, but may be an indentation or well in the wheel **400** for holding the bag **35** to be irradiated. If this is the case, **401** may be made of material which is transparent to radiation, and may thus be made of a transparent polycarbonate, plexiglass, quartz glass or other sturdy substantially transparent material to allow light transmissivity therethrough. It is also contemplated that the indentation or well **401** may be made of material which is not transparent to radiation. The bag **35** may be clamped or otherwise held in place at four corners and/or along the sides at one or more locations with any of numerous alternative devices (not specifically shown). However, at least one bar or clamp device **470** is preferably used to provide the narrowed flow constrictions described above for the enhancement of mixing.

Preferably, as shown in Fig. 13, a bag **35** is disposed in the wheel **400** and constrained between two cross bar clamping members **471, 472** to squeeze or narrow the dimension therebetween for mixing. These may be disposed to divide the chamber of the container into approximate half or other sized portions. Then the wheel **400** is preferably disposed in an operably rotatable manner in a device (not shown) which will provide the rotating force to rotate the wheel **400** about its central axis **480** (Fig. 13). In one embodiment, the rotation will be ± **360** continuous degrees with light shining at the bag from both sides of the bag/wheel. In one embodiment, the wheel may be rotated in an alternating fashion in one direction for a period of time, and then the wheel could be reversed to rotate in the other direction for a period of time. Gravity is used to first pull the contents of the bag **35** down into the lower portion of the bag **35,** and as the bag is rotated and turned or flipped over, gravity pulls the contents down into what was the upper portion, now inverted and disposed under the previous lower portion (Fig. 6). Upon continued rotation, the bag is re-inverted so that the original upper portion is once again disposed above the original lower portion and gravity pulls the contents again down into this lower portion (Fig. 6). As shown in Fig. 6, upon each inversion, the flow of contents passes through a constriction **55** between clamping members **471, 472** *et al*. and creates mixing as depicted. Such mixing again provides for scrubbing the inner bag surface to ensure removal of already irradiated material therefrom and ensures that new material not yet irradiated is deposited there so that the newly deposited material may be exposed to light.

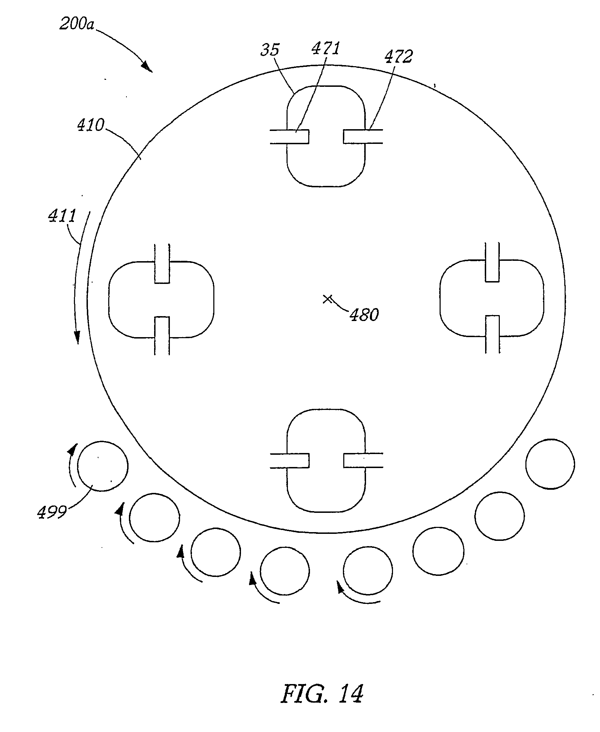

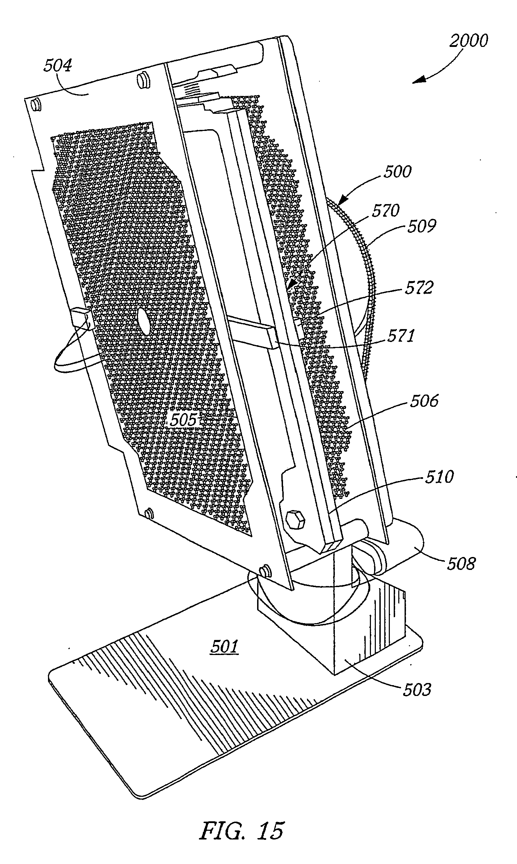

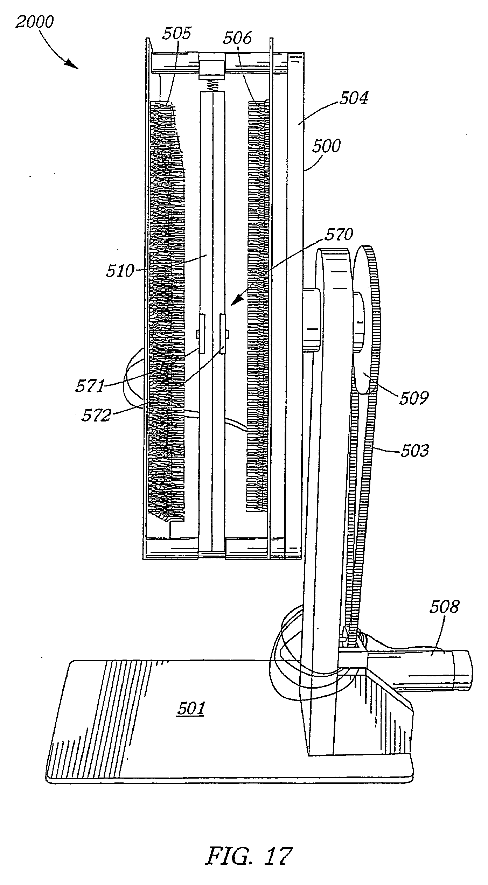

Another version 200a of a wheel 410 is shown in Fig. 14 in which multiple (here, four (4)) bags 35 are shown disposed thereon for rotation with the wheel 410. Clips or clamps **471, 472** are also shown for holding the bags and/or providing the flow constrictions for mixing as described throughout. Rollers **499** are shown schematically as one alternative for providing the motion necessary to rotate wheel 410 (or 400, see Figs. 12, 13). Thus, such rollers could be disposed in a device (not shown but not unlike that shown in Figs. 10, 11) and be mechanized to roll in a direction similar to that shown in Fig. 14 to provide a rotational motion to the wheel 410 designated by the arrow 411 in Fig. 14, and thus the wheel and bag(s) may be rotated and the contents thereof mixed while also being exposed to photoradiation. Note, though not shown in these rotating wheel embodiments, light sources may be used to shine light on one or the other, or in some embodiments, both sides of a rotating bag for photoactivation of photosensitizer chemicals in the bag, if used. Such lights may be disposed in banks on either side of the respective bag. These banks may thus be stationary relative to the rotating/moving bag (see Fig. 10) and/or wheel device such as wheel 400 (or 410, inter alia), or the light banks may be made to rotate with the bag. As an example of this please see the further alternative wheel embodiment as shown in the embodiments of Figs. 15-18.

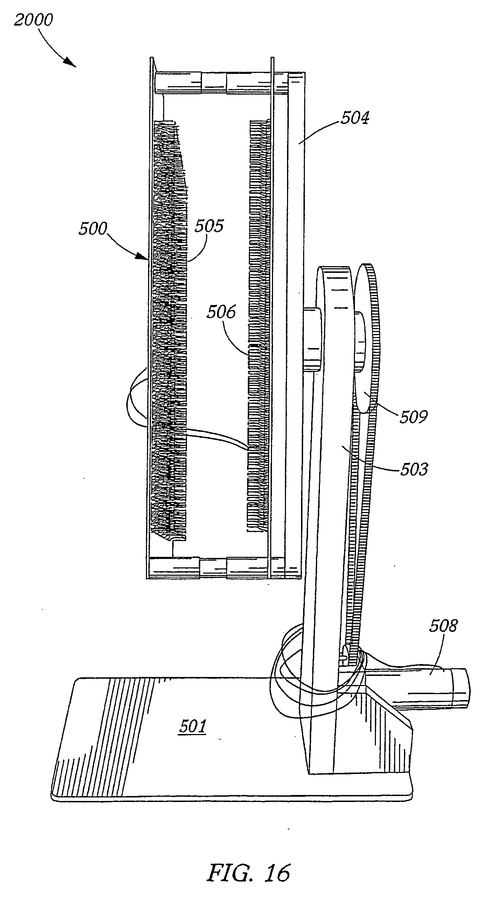

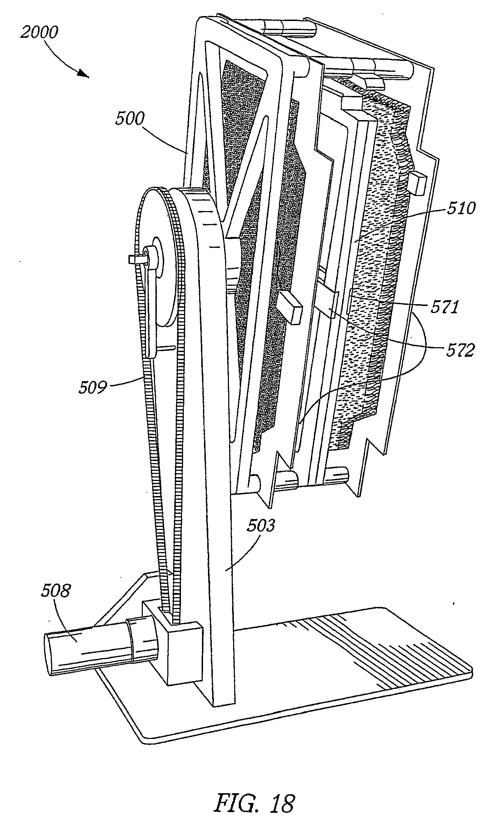

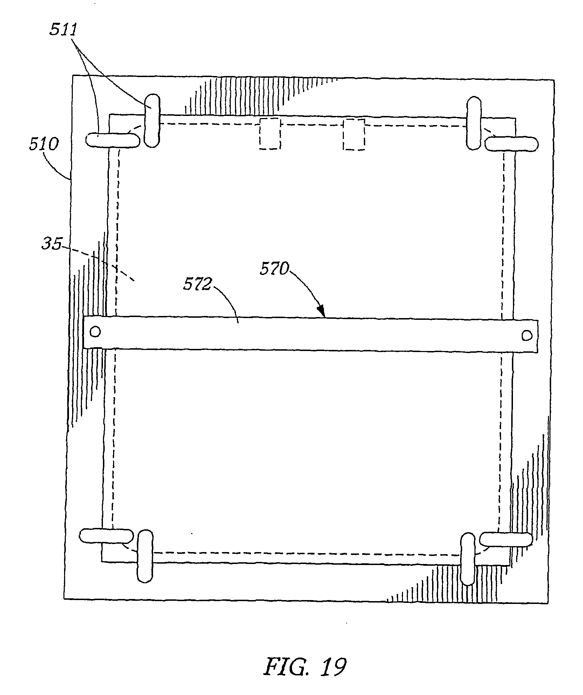

In the embodiment of Figs. 15-18, a system **2000** is shown with a device **500** which includes a base **501,** a support arm **503** and a rotatable member **504** which here includes front and back light banks **505, 506** which are thus rotatable. A motor **508** with a chain link style gear driving mechanism **509** is also show in Figs. 15-18 (particularly Fig. 18). A frame **510** which is adapted to hold a bag 35 (as shown in Fig. 19, see below) is shown as disposed in device 500 in Figs. 15,17 and 18 (Fig. 16 shows device 500 without frame 510). Thus, when a bag **35** is disposed in a frame (see Fig. 19), and frame **510** is disposed in device **500,** the motor 508 can be activated to turn the gear assembly 509 which in turn rotates the rotatable member **504** with light banks **505, 506** and frame **510.** A mixing action of contents in bag **35** like that shown and described relative to Fig. 6 then occurs.

As shown in more detail in Fig. 19, a bag **35** (shown in dashed lines), held in place by clips/clamps/hooks **511,** may also be crossed by a crossbar clamping assembly **570** which includes a cross bar member **572** which provides a flow constriction for mixing like that shown in Fig. 6. It should be noted that constriction element **570** extends the entire length of the bag, however, constriction element **570** may also extend partially across the bag such as the constriction elements shown in Figs. 3 and 13. A corresponding bar **571** is shown in Figs. 15, 17, and 18 on the opposing side of the frame **510.**

Thus, when rotated and illuminated, a pathogen reduction process can be achieved. In this embodiment, the irradiation source is shown as being LEDs (light emitting diodes). One advantage in using LEDs is their ability to be located in close proximity to the bag containing fluid to be pathogen reduced without emitting much heat, which could damage the blood or blood component being irradiated. LEDs are also useful in this invention because they emit light in very narrow bandwidths. Light in a narrow spectrum may be beneficial to the blood product being irradiated because all non-useful wavelengths of light which might damage the blood or blood components are eliminated.

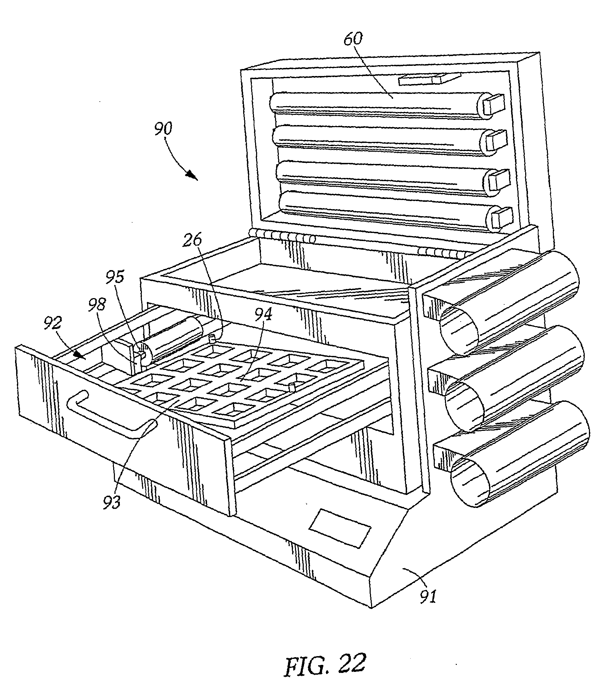

Fluorescent bulbs may also be used as the irradiation source (see Figs. 10 and 22). Visible or ultraviolet light may be used, depending on the type of blood or blood product to be pathogen reduced as well as the type of photosensitizer to be used, if any.

Note, in the rotatable examples, more resilient or even rigid containers may be used. Constriction elements which are built in or completely removable may also be used in these embodiments.

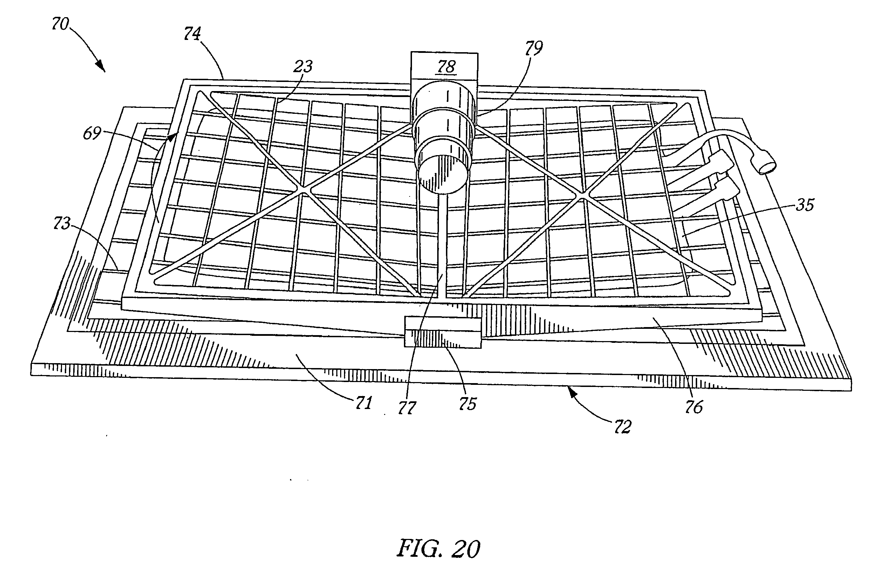

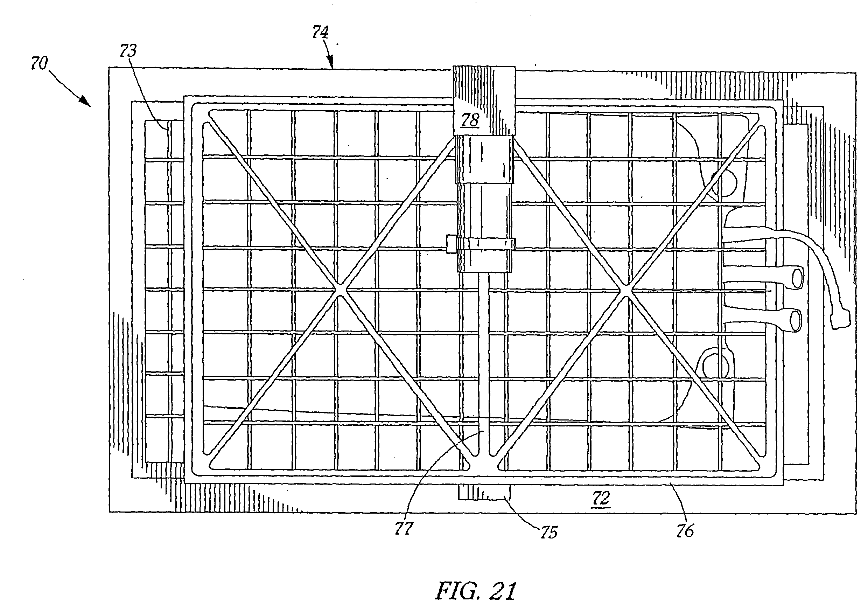

Further embodiments may include other than the substantially vertical embodiments described heretofore (which take advantage of gravity as a flow forcing element therein). For example, substantially horizontal-mixing structures may also be used. A first such embodiment is shown in Figs. 20, 21. The system **70** of Figs. 20, 21 includes a mixing device **72** which may include a base frame **71,** to and/or in which may be a screen-like support **73** on which a bag **35** may be disposed (note, metal or transparent plastics may be used). A rotatable squeezing or clapping assembly **74** may then be disposed in operative relationship therewith. Assembly **74** is connected via a pivot assembly **75** to the frame **71** assembly in relative central position and preferably has one or two flappers **76** which may be moved down onto and squeeze the bag **35** when this is disposed therein. A motor **78** may be used to impart the back and forth rotational movement, through motor connection **79,** to the rotatable assembly **74.** Back and forth rotation hereof is generally shown by the arrows **69** (Fig. 20.) A constriction or clamp member **77** may also be used herewith. Indeed, constriction member **77** may be a part of the rotation connection assembly, as for example, an axle which may lay across the bag **35,** again in a substantially central location. A corresponding upraised portion or member (not directly shown) may be disposed on the underside of bag **35** as perhaps a portion of or a member connected to the support structure **73.** A further alternative in this or any of the embodiments in this specification, is to use a separate clamping assembly (not separately shown), not directly attached to the device **72.** In any case, it may be preferred (though not necessary) to have a constriction device in order to provide the mixing action shown and described relative to Fig. 6 above.

Note, as introduced above this system **70** can be disposed substantially horizontally, so long as there is access to lights if so desired, and in an embodiment these could be shining from both top and bottom sides (although one side or the other may also be operable). Even so, it could also be disposed vertically or in numerous other dispositions in three-dimensional space.

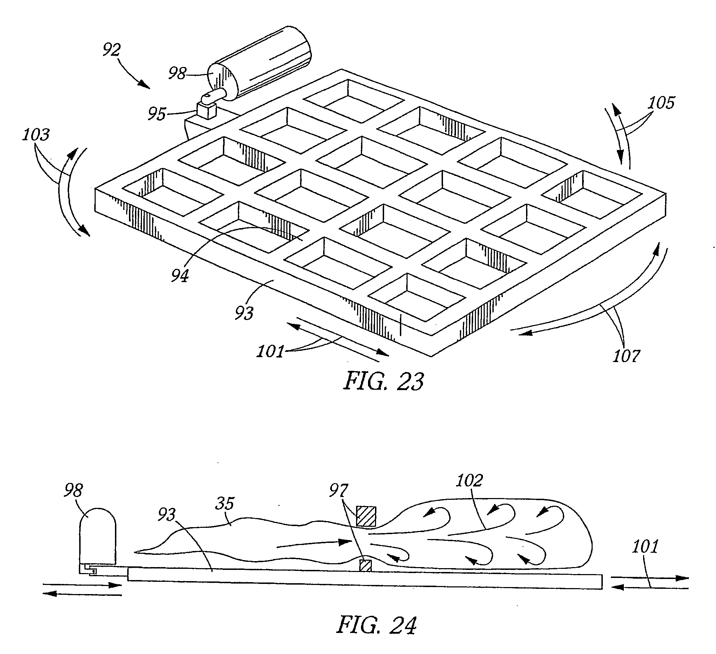

A further usually substantially horizontally disposed embodiment is shown in Figs. 22, 23, 24 and 25 (although this also could be disposed in any number of other dispositions in 3D space). In the primary embodiment shown in Fig. 22, the system **90** is shown which includes an irradiation unit **91** in which is disposed a mixing device **92.** Device **92** includes a moveable tray or frame **93** with a screen or other support **94.** As shown in more detail in Fig. 23, the movable tray **93** may be connected by a pivot assembly **95** to a motor **98** which may provide the movement for mixing the contents of the bag 35 which may be disposed thereon (see Figs. 24 and 25, described below). In one embodiment, a clamp device **97** (see Figs. 24-25) may be also included to provide flow-mixing vortices as shown and described both above and below.

As shown in Fig. 24, the moveable tray **93** may be moveable back and forth in a lateral or longitudinal type of motion or both. This is shown by the lateral arrows **101.** This can then create the fluid flow vortices identified by the flow arrows **102** inside bag **35.**

Similarly, as shown in Fig. 25 the tray may also be rotated slightly to cause fluid motion in the bag **35.** This rotation is shown by arrows **103** and the fluid flow vortices created thereby are shown by arrows **104** in the bag **35.** Note, the tray **93** may rotate in either or both lateral or longitudinal rotations (e.g., rotations about lateral or longitudinal axes). Lateral rotations are shown by arrows **105** (Fig. 23.) The movements such as the back and forth lateral or longitudinal movements (as shown in Fig. 24) may be combined with rotations in other directions (as in the lateral and/or longitudinal rotations as shown in Fig. 25), and may be in either ordered or random combinations. Thus wobbles and/or nutations (as from wobbulators or nutators, as known) may be further alternatives included here as well. Still further rotations may be circular or elliptical or other orbital movements, as depicted generally by the arrows **107** in Fig. 23. Such orbital motions may be used in lieu of or in addition to those previously described. Churning, undulating, oscillating, gyrating, shaking and/or stirring are but a few of a host of other motions which may be performed here within one, two or three dimensions, alone or in combination to provide mixing according to this invention.

Fig. 26 shows another embodiment of a flexible container which may be used in/with the present invention (squeezing or rotation, or mere movement) wherein the flow constriction assembly is a part of the bag/container **350.** The container **350** may be made of polymeric type film material extruded in a tube-like shape. The main body compartment of container **350** has two partial seals or welds **370** which at about half way up the length of the main body compartment extend inwardly from the sides of the container **350.** The partial seals or welds **370** divide the main body portion into two partial sub-compartments **351, 352** respectively. Although shown as extending inwards from the sides of container **350,** one or more partial seals or welds **370** may extend from any of the four sides of the bag **350** without departing from the spirit and scope of the invention. These partial seals or welds **370** are intended to provide vortices **58** in the fluid during the mixing process.

Fig 27 shows a plan view of another alternative embodiment of a further polymeric container **3500** which may be used in/with the present invention. This container **3500** may be made of a polymeric type film such as PVC or polyolefin (flexible or not). The container may be sealed or welded around its outer border zones during manufacture. The seals or welds create a fluid tight, sealed interior space. The container is configured in a substantial figure eight or hourglass shape as shown (and as introduced above). The container **3500** has an upper expanded interior or sub-compartment portion **3510,** and a lower expanded interior portion or sub-compartment **3520.** The upper expanded sub-compartment **3510** and the lower expanded sub-compartment **3520** are connected to each other in a fluidly communicative relationship by narrowed portion **3555** defined by the indented sides of the container. Both the upper expanded sub-compartment **3510** and the lower expanded sub-compartment **3520** may also be further sub-divided into multiple sub-compartments (not shown) without departing from the spirit and scope of the invention. As shown in Fig. 27 a constriction **3700** is formed by the figure eight or hourglass shape in such a fashion so as to divide bag **3500** into two separately contained fluid-tight or fluidly separated sub-compartments **3510, 3520.** Sub-compartment **3510** is located above sub-compartment **3520.**

As shown in Fig. 27, the figure eight or hourglass configuration may aid in mixing the solution within the bag **3500.** The fluid in the bag may be mixed in a substantially vertical manner or a substantially horizontal manner and/or any angle therebetween (although a vertical disposition will have gravity assistance). A force imparted to the bag at a particular location creates the movement of fluid within the bag. As the fluid is forced to move through the narrowed portions 3555 of the hourglass/figure eight shape, vortices 58a are created within the fluid which helps to further mix the fluid. A clamp (not shown) such as those described herein may also be used to create narrowed portions within the bag. A seal or weld like a clamp structure could also be used to further separate any portion into two smaller sub-compartments and may thus also assist in creating further vortices within the fluid. The narrowed portion or portions create vortices within the fluid as the fluid is forced to flow through the narrowed portions as described throughout.

Fig. 27 shows the bag **3500** in the figure eight or hourglass configuration being mixed by clapping or rotation in either a substantially vertical and/or a substantially horizontal manner. If rotated, the bag may be rotated about its centerpoint (not shown) between about 150° and about 360° in a continuous fashion. A force (from the rotation itself and/or due to the force of gravity when in a vertically disposed embodiment) may be imparted to the bag by virtue of the rotation of the bag to create the initial movement of fluid within the bag. As the fluid is forced to move around the figure eight or hourglass shape, vortices are created within the fluid which helps to further mix the fluid. The bag may be rotated in a continuous manner, or may be agitated in varying degrees from between about 0° to about 360° without departing from the spirit and scope of the invention. The bag may also be rotated either singly or in a repetitive manner.

The examples of the above-described systems, methods, and apparatuses and bags are for illustrative purposes only. Because of variations which will become apparent to those skilled in the art, the present invention is not meant to be limited to the particular embodiments described above. Any such variations and other modifications or alterations are included within the scope and intent of the invention.

The claims of the parent application are reproduced below. These are not the claims of the present divisional application which appear in the separate section headed "Claims".
1. A mixing system for use in mixing a fluid contained within a fluid container, the system comprising:
   a support structure;
   at least one movable squeezing element operably disposed relative to the support structure;
   a holding device adapted for holding a fluid container between the support structure and the movable squeezing element;
   an irradiation source for irradiating the fluid contained within the fluid container; and
   whereby the movable squeezing element is adapted to move so as to squeeze the fluid container against the support structure to thereby mix the fluid contained in the fluid container.
2. The mixing system according to claim 1 wherein the irradiation source irradiates the fluid while the fluid contained in the fluid container is thereby mixed.
3. A mixing system according to claim 1 in which the fluid to be mixed is whole blood.
4. A mixing system according to claim 1 in which the fluid to be mixed is a blood component.
5. A mixing system according to claim 1 in which the fluid to be mixed includes a pathogen reduction agent.
6. A mixing system according to claim 5 in which the pathogen reduction agent is a photosensitizer.
7. A mixing system according to claim 6 in which the photosensitizer is riboflavin.
8. A mixing system according to claim 6 in which the photosensitizer is a psoralen.
9. A mixing system according to claim 1 which further comprises a motor which is operably connected to the movable squeezing element to move the movable squeezing element.
10. A mixing system according to claim 9 in which the motor moves the movable squeezing element in a reciprocal motion, closed and open to alternately squeeze and release the fluid container.
11. A mixing system according to claim 1 in which the movable squeezing element is rotatable about a pivot point.
12. A mixing system according to claim 1 which further comprises a constriction element disposed in operative relationship with the fluid container.
13. A mixing system according to claim 1 in which the fluid container is a flexible bag.
14. A mixing system according to claim 1 in which the fluid container is a substantially resilient container.
15. A mixing system according to claim 12 in which the constriction element provides a narrowed fluid flow passage within the fluid container which thereby provides a vortex action of fluid being moved therein.
16. A mixing system according to claim 12 in which the constriction element is a clamp.
17. A mixing system according to claim 16 in which the clamp is removably connected to the support structure.
18. A mixing system according to claim 16 in which the clamp is permanently affixed to the support structure.
19. A mixing system according to claim 12 in which the constriction element is a feature of the container.
20. A mixing system according to claim 19 in which the feature of the container is a welded seam.
21. A mixing system according to claim 19 in which the feature of the container is a side indentation.
22. A mixing system according to claim 1 in which the irradiation source provides light to at least two sides of the fluid container.
23. A mixing system according to claim 1 in which the fluid container is held in a substantially vertical orientation.
24. A mixing system according to claim 1 in which the fluid container is held in a substantially horizontal orientation.
25. A mixing system for use in mixing a fluid contained in a fluid container, the system comprising:
   a rotational support structure;
   an irradiation source for irradiating the fluid contained in the fluid container;
   a holding device disposed in operable relationship with the support structure;
   whereby the holding device is adapted to hold the fluid container; and
   whereby when the fluid container is disposed in the rotational support structure;
   whereby the rotational support structure may be rotated to thereby rotate the fluid container, and thereby mix the fluid contained therein.
26. The mixing system according to claim 25 wherein the irradiation source irradiates the fluid while the rotational support structure is rotated to mix the fluid contained in the container.
27. A mixing system according to claim 25 in which the mixing system further includes:
   a roller housing; and
   at least one roller mechanism disposed in said roller housing; whereby the rotational support structure is adapted to be disposed in operable rotational engagement with the at least one roller mechanism such that at least one roller mechanism imparts a rotational movement to the rotational support structure.
28. A mixing system according to claim 27 in which the mixing system further: includes:
   a motor disposed in operable relationship with the at least one roller mechanism;
   whereby the motor is adapted to impart a rotational movement to the rotational support structure.
29. A mixing system according to claim 28 in which the mixing system further includes:
   a motor; and
   a motor connection assembly disposed in operable relationship with the motor; and
   whereby the motor connection assembly is disposed on operable rotational drive relationship with the rotational support structure.
30. A mixing system according to claim 25 which further comprises a constriction element disposed in operative relationship with the fluid container.
31. A mixing system according to claim 25 wherein the irradiation source further comprises LEDs.
32. A mixing system according to claim 25 wherein the irradiation source further comprises fluorescent bulbs.
33. A mixing system according to claim 25 wherein the irradiation source emits light in the visible region.
34. A mixing system according to claim 25 wherein the irradiation source emits light in the ultraviolet region.
35. A mixing system according to claim 25 which the fluid to be mixed includes a blood product.

## Claims

1. A mixing system for use in mixing a fluid contained in a fluid container, the system comprising:
a lateral support structure;
a double sided movable squeezing element operably disposed relative to said lateral support structure;
an irradiation source; and
whereby said fluid container contains a fluid to be mixed; and
whereby the double sided movable squeezing element is adapted to move so as to squeeze the fluid container against said lateral support structure to thereby mix the fluid contained in the fluid container.

2. A mixing system for use in mixing a fluid, the system comprising:
a lateral support structure;
an irradiation source;
a holding device disposed in operable relationship with the lateral support structure; whereby the holding device is adapted to hold a fluid container;
whereby the fluid container contains a fluid to be mixed; and
whereby when a fluid container is disposed in said lateral support structure, said lateral support structure may be moved to thereby move the fluid container, and thereby mix the fluid contained therein; and
whereby the irradiation source irradiates the fluid in the fluid container while the fluid container is being mixed.

3. A mixing system according to claim 2 in which the lateral support structure is laterally moveable to laterally move the fluid container.

4. A mixing system according to claim 2 in which the lateral support structure is rotationally moveable to rotationally move the fluid container

5. A mixing system according to claim 4 in which the rotational movement is about a transverse axis.

6. A mixing system according to claim 4 in which the rotational movement is about a longitudinal axis.

7. A mixing system according to claim 2 in which the lateral support structure is circularly moveable to circularly move the fluid container

8. A mixing system according to claim 2 in which the lateral support structure is elliptically moveable to elliptically move the fluid container

9. A mixing system according to claim 2 in which the fluid to be mixed includes a blood product.
